# EUROPEAN PATENT SPECIFICATION

(11) **EP 1 919 573 B1**
(45) Date of publication and mention of the grant of the patent: **01.08.2012**
(21) Application number: 06795221.8
(22) Date of filing: 08.08.2006
(51) Int. Cl.: A63B 24/00, A63B 71/06

(54) **PERFORMANCE MONITORING APPARATUS**
LEISTUNGSÜBERWACHUNGSVORRICHTUNG
APPAREIL DE SURVEILLANCE DES PERFORMANCES

(30) Priority: 08.08.2005 CN 05106805
(43) Date of publication of application: 14.05.2008
(73) Proprietor: Dayton Technologies Limited, Hong Kong SAR (HK)
(72) Inventor: YUEN, Paul, Anthony, Hong Kong SAR (CN)
(74) Representative: Simcox, Michael Thomas
(86) International application number: PCT/IB2006/002166
(87) International publication number: WO 2007/017739

(56) References cited:
- EP-A- 1 512 370
- WO-A-2004/058059
- WO-A-2005/082471
- WO-A1-2005/082472
- US-A1- 2005 129 253
- US-A1- 2006 046 905
- US-B1- 6 230 047

## Description

### FIELD OF THE INVENTION

This invention relates to monitoring apparatus for monitoring physical or physiological parameters to determine performance information of a person, and more particularly, to monitoring apparatus for monitoring physical or physiological parameters of a person during physical exercises.

### BACKGROUND OF THE INVENTION

Physical or physiological parametric performance information, for example, body cadence information, can provide useful information on the physical conditions of a person. For example, heart-rate is an important parameter which provides useful body cadence information on the physical fitness and performance limit of a person. Heart-rate monitors, for example those with chest strap sensors or finger touch sensors, are commonly available to assist a user to monitor his/her heart-rate during physical exercises so that a user can ensure that the exercise heart-rate is within a desirable range for achieving optimal objectives. As a specific example, fitness equipment is commonly provided with heart-rate zone indicators so that a user can choose to exercise in one of a plurality of zones, for example fat burning, aerobic or cardiovascular exercise. More importantly, heart-rate monitors provide a useful guide to an exerciser so that the maximum allowable safe heart-rate is not exceeded.

Other useful body cadence information include, for example, foot-stroke span, period or frequency, arm-stroke span, period or frequency, cycling cadence and/or other parameters, are useful to assist appraising of performance during physical exercise.

Such physical parametric performance information, or more specifically, the body cadence information, is commonly used to evaluate the performance of a person during physical exercise. For example, an exerciser can set various performance targets or objectives to be achieved during physical exercises. Furthermore, an exerciser may like to cover a pre-determined distance within a pre-determined time by running, jogging or swimming. On the other hand, some exercisers may like to maintain a particular speed or follow a particular cadence pattern during physical exercise to meet their specific objectives. Such performance can be evaluated through monitoring of various types of body cadence information. In general, body cadence information is rhythmic and/or cyclical.

Conventional performance monitoring devices for monitoring physical (e.g. speed, stroke) or physiological (ECG) parameters and relevant performance information are typically equipped with a visual information display through which the relevant physical performance information, including body cadence information, can be viewed for reference. However, use of such conventional monitoring devices almost inevitably requires regular, or at least intermittent, interruption or disruption of a user from the regular exercise pattern or exercise modulus.

As a convenient example, heart-rate monitors are commonly worn by sports enthusiasts so that their instantaneous heart rates can be monitored. Such heart-rate monitors usually come in the form of chest-strap or finger-touch type with a wrist-worn visual display (VDU). When the heart-rate information is to be read from the VDU, an exerciser will have to slow down the pace or even make a short pause in order to operate the heart rate monitor for display of information. Such an interruption during sports is often regarded as inconvenient, if not a nuisance or dangerous, and is desirable.

Therefore, it will be beneficial if cadence monitoring apparatus which mitigate shortcomings of known performance monitoring apparatus can be provided.

In this specification, the term cadence information or body cadence information is broadly used to cover physical cadence of a person, including but not limited to heart rate, foot-stroke span, period or frequency, arm-stroke span, period or frequency, cycling cadence and/or other parameters which are rhythmic or cyclical. In addition, the term "physical or physiological parameter performance signals" is broadly used to cover signals of a person which are generated during physical exercise. Such signals include both physical signals of a user, such as ECG signals, and other performance signals generated by a sensor or a signal transducer during physical exercise. For example, such performance signals comprise signals generated by foot-strokes of a user during jogging or running, arm-strokes generated during running or swimming, cycling cadence and other sports related performance signals. Such performance signals are typically cyclical and rhythmic.

EP 1512370 discloses a training control method and apparatus using biofeedback are provided. The training control apparatus using biofeedback, comprises a bio-signal measuring module measuring a bio-signal from a user and providing predetermined biofeedback information to the user; and a bio-information feedback module setting a target exercise zone and a target resting zone using the bio-signal received from the bio-signal measuring module and user information, comparing the bio-signal with either of the target exercise zone and the target resting zone according to a training mode, and providing one of positive biofeedback information and negative biofeedback information in the selected training mode to the user according to a result of the comparison.

Therefore, it is desirable if there can be provided an improved cadence monitoring apparatus which at least partially mitigates some of the shortcomings of conventional performance monitoring apparatus of the prior art.

### SUMMARY OF THE INVENTION

According to the present invention, there is provided a claim 1.

According to another aspect of this invention, there is provided claim 28.

The output feedback signal is preferably in the form of physical stimulation, for example, as audio, music, physical or electrical beats, audio announcement of the instantaneous parameters or the required trends.

By providing a feedback by means of physical stimulation, a user can be informed of his/her instantaneous performance without looking at a VDU so that the user can determine on the subsequent level of activities without significant disruption or distraction form the normal modulus of exercise.

Advantageously, the apparatus further comprises a feedback signal source for generating said output feedback signal, wherein the contents and/or rhythm of said output feedback signals are dependent on the outcome of comparison of said cadence comparison means. With such an audio feedback scheme; a user is informed of the user's current performance status so that the user can determine on the next desirable cadence without visually referring to a VDU

In a preferred embodiment, at least a stream of audio signals of a pre-determined rhythm is stored in said feedback signal source, said stream of pre-stored audio signals being for constituting a stream audio output feedback signals for delivery to a user. The stream of audio signals can serve as a reference signal output so that a user can react intuitively upon hearing a variation thereof.

Preferably, the rhythm of said audio output feedback signals is varied from said pre-determined rhythm, departure of said body cadence signals from said preset or predetermined reference cadence level is represented by the extent of variation of said rhythm of said audio output feedback signals from said pre-determined rhythm. Such a feature will enable a user to react intuitively with reference to the extent of departure from the reference rhythm in order to adjust the performance cadence to meet the desired level.

In one embodiment, the rhythm of said audio output feedback signals is dependent on the outcome of comparison between said detected body cadence signals and said reference cadence level or said reference cadence levels.

Advantageously, said output feedback signals further comprises audio information relating to instantaneous body cadence information of a person. With the audio information, a user can be informed of the instantaneous body cadence information without reference to a VDU.

In a preferred embodiment, signals stored in said feedback signal source has a pre-determined rhythm, the rhythm of output feedback signals for delivery to a user being variable from said pre-determined rhythm such that the extent of variation of the rhythm of said feedback signals from said pre-determined rhythm is representative of the extent of departure of said body cadence signals from said preset or predetermined reference cadence level.

Specifically, although not limiting thereto, said cadence information comprises heart rate, foot-stroke frequency, hand-stroke frequency, lap time (swimming), running speed, and like body parametric performance information.

As an example, the instantaneous rhythm of said output feedback signals is decreased from said pre-determined rhythm to a lower rhythm when said detected cadence signals is above a threshold cadence level.

As a further example, said feedback signal source comprise background audio output signals and cadence information audio output signals, said background audio output signals being delivered continuously to a user and said cadence information audio output signals being delivered to a user intermittently.

Preferably, said cadence information audio output signals are delivered to a user at regular intervals, at pre-determined intervals and/or upon detection of a variation of the cadence information exceeding a pre-determined range.

For example, said cadence information audio output signals are delivered when delivery of said background audio output is interrupted.

Advantageously, delivery of background audio output signals and delivery of cadence information audio output signals share a common audio channel.

As an example, said background audio output comprises rhythmic audio signals such as songs, music, temporal or rhythmic beats, or the like.

Preferably, rhythm of said background audio output is varied according to the instantaneous body cadence information. This gives a user an instantaneous feedback of the user's instantaneous performance.

Preferably, said feedback signals comprise audio delivery of body cadence information in numerical values.

Preferably, said sensor is for sensing cadence parameters of a person arising from physical exercises, said controller comprising processing means for converting said cadence parameters into performance indicating parameters.

For example, said performance indicating parameters includes heat rate, swimming speed, lap time, running speed, blood pressure, or like parameters.

Preferably, said sensor is adapted for sensing at least one of the following parameters: stroke frequency (including foot-stroke frequency and arm-stroke frequency), stroke period (including foot-stroke period and arm-stroke period), pedalling frequency, pedalling speed, running speed. Advantageously, the performance indicating parameters comprise running speed, swimming speed, cycling speed, expected time to complete a prescribed range or the like.

Preferably, the sensor is for sensing pedalling frequency of a cyclist and the feedback signals comprises at least one of the following numerical values-cycling cadence, cycling speed, remaining time-to-arrive or like information.

Advantageously, said apparatus further comprising an ambient sensor for sensing at least one of the following ambient parameters: wind speed, wind direction, temperature, UV level, humidity; wherein said audio output signals further comprises at least one of the following numerical values- wind speed, wind direction, temperature, UV level, humidity.

Preferably, the sensor is for sensing step frequency of a runner and the audio output signals comprises at least one of the following numerical values-running cadence, running speed, remaining time-to-arrive or like information.

Advantageously, said apparatus further comprising storage means for storing at least one target cadence value or at least on target performance value, wherein said cadence comparison means comprises means for comparing detected cadence information against said target cadence value, and said controller generates an audio feedback to a user regarding the instantaneous performance of a user.

Preferably, said audio feedback comprises musical beats indicative of the rhythm of exercise that a user is required to perform in order to achieve, reach or maintain said target cadence value or said target performance value.

Preferably, said feedback source comprises a background audio source for generating background audio signals, said background audio source comprising a radio receiver, a MP3 player, or other digital audio playback devices.

Preferably said sensor is housed in a first housing and said audio source is housed in a second housing, said first and second housing being detached from each other, and said sensor and said control means being wirelessly linked.

Advantageously, said apparatus further comprising wireless communication means for transmitting said detected performance related cadence information wireless.

### BRIEF DESCRIPTION OF THE DRAWINGS

Preferred embodiments of the present invention will be explained in further detail below by way of examples and with reference to the accompanying drawings, in which:-
Fig. 1 is a block diagram of a monitoring apparatus of the present invention,
Fig. 2 is a flow chart showing an exemplar Fig. 1,
Fig. 3 shows a perspective view of an output device of the apparatus of Fig. 1,
Fig. 4 shows a front view of the apparatus of Fig. 3 during use,
Fig. 5 shows a schematic block diagram of a feedback device of the apparatus of Fig. 1,
Fig. 6 shows an exemplary application of an apparatus of this invention,
Fig. 7 shows another exemplary application of the apparatus of this invention, and
Fig. 8 shows another preferred embodiment of this invention.

### DETAILED DESCRIPTION OF THE PREFERRED EMBODIMENTS

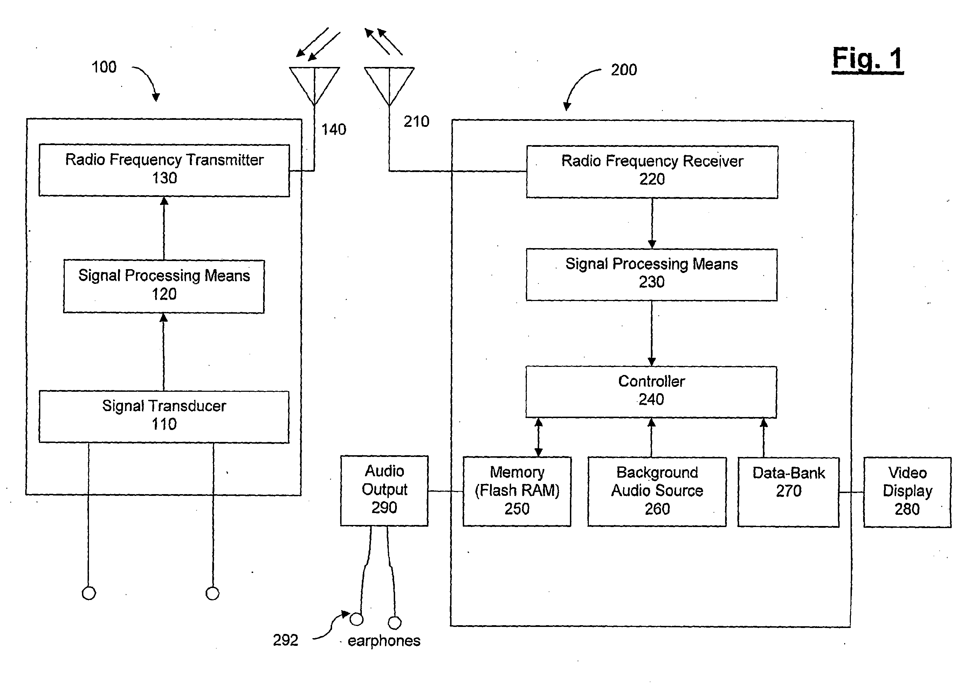

Schematic arrangements of a monitoring apparatus of the present invention are shown in Fig. 1. The monitoring apparatus comprises a sensing device **100** and an output device **200**. The sensing device **100** is for monitoring physical or physiological parameters, especially body cadence parameters, of a person. Typical physical or physiological parameters include, for example, ECG pulses, cycling cadence, stroke frequency (hand or foot).

The sensing device **100** comprises a .signal transducer **110**, signal processing means **120** and a radio-frequency transmitter **130**. The signal transducer **100** comprises a sensor for detecting physical or physiological information of a person. The physical information to be detected includes, for example, ECG pulses, spans, foot-stroke frequency, foot-stroke periods, foot-stroke spans, arm-stroke frequency, arm-stroke periods, cycling cadence and other like physical cadence parameters. The sensor can be a motion cadence sensor or an ECG sensor. Motion cadence sensors or ECG sensors are staple items which are readily available as module forms or as discrete components.

The signal transducer **110** is for converting the detected physical or physiological parameters into electrical signals for further processing. Where the sensor comprises an ECG sensor, the signal transducer **110** typically comprises a pair of electrodes for making physical and differential electrical contact with the body of a user, and an amplifier for amplifying the detected ECG signals. Where the sensor comprises a motion cadence sensor, the signal transducer **110** typically comprises means for converting physical motion, especially cyclical motion, into electrical signals.

Electrical signal outputs of the signal transducer are then processed by the signal processing means **120** for transmission by the radio-frequency transmitter **130** via an antenna **140**. The baseband output signals of the transducer are modulated and/or encoded by the signal processing means **120**. The radio frequency transmitter **130** and the signal processing means **120** together prepare and transmit the baseband signals at 2.4 GHz according to Bluetooth^{®}, ANT, or other standards.

The output device **200** comprises an antenna **210** for receiving the radio signals transmitted by the sensing device **100**, a radio frequency receiver **220**, signal processing means **230** and a controller (CPU) **240**. The output device **200** comprises a radio frequency receiver **220** for detecting and processing the wireless signals transmitted by the detector, signal processing means **230** to convert (for example, to demodulate and decode) the received radio frequency signals to base-band signals which can be readily processed by the controller **240**. The controller comprises a micro-controller, e.g. a micro-processor, which has been programmed to process the received baseband information and to generate outputs according to a set of prescribed criteria to be described below.

After the incoming signals which carry the relevant body cadence information or other information have been processed at the controller 240, the controller will cause the relevant physical or physiological information to be displayed on a VDU (video display unit) or to be delivered through audio means, for example, through a pair of ear phones to a user.

The output device **200** further comprises a storage means **250**, for example, flash RAM, a background audio source **260**, such as a MP3 or other digital audio source, and a databank **270** for providing data necessary for synthesizing an audio output which carries the desired body cadence information.

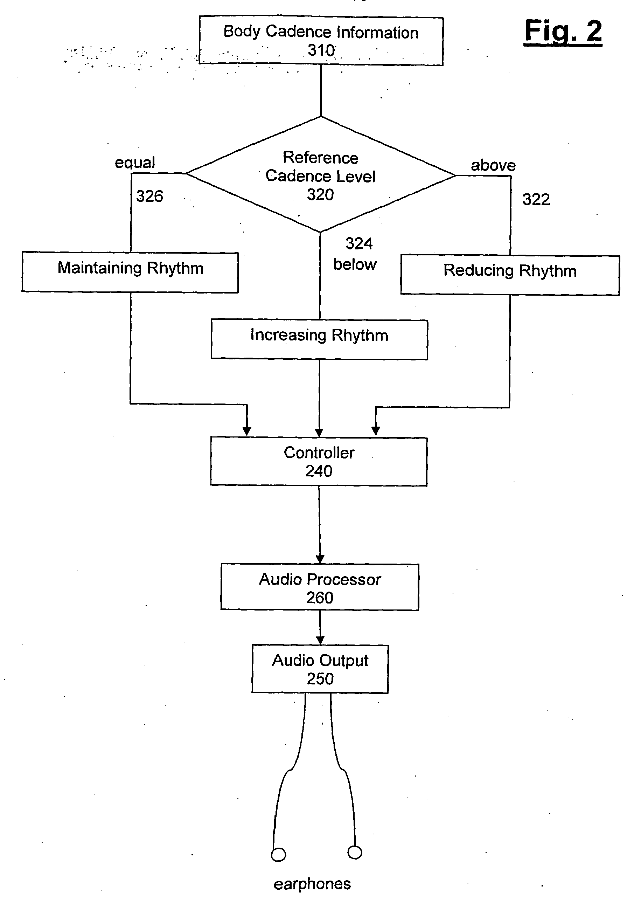

Referring to Fig. 2, there is shown a hybrid flow chart and block diagram illustrating an exemplary operation of the invention. When a stream of data representing body cadence information **310** arrives at the controller **240**, cadence comparison means of the pre-programmed controller will compare the body cadence information **310** against at least one pre-determined reference cadence level **320**.

For example, when the body cadence information **310** carries a stream of ECG pulses, the reference cadence level **310** can be set to represent a maximum allowable heart rate, a minimum allowable heart rate, a preferred range of heart rates, or other preferred heart rates.

The output of the cadence comparison means will depend on the outcome of comparison between the detected body cadence information **310** and the reference cadence level. For example, if the received body cadence information **310** has exceeded the reference cadence level, for example, the maximum allowable, heart rate, an "above reference" signal **322** will be output. If the received body cadence information **310** is below the reference cadence level, for example, the maximum allowable heart rate, a "below" signal **324** will be output. On the other hand, if the received body cadence information **310** is equal to the reference cadence level, or within a reference range, an "equal" signal **326** will be output.

As another example, the body cadence information **310** can carry motion cadence information, for example, cycling cadence, stroke frequency, stroke period etc. In this example, the reference cadence level **310** can be set to represent a maximum allowable speed, rate, frequency, or period, a minimum allowable speed, rate, frequency, or period; a preferred range of speed, rate, frequency, or period; or other preferred speed, rate, frequency, or period.

In such an application, if the received body cadence information **310** has exceeded the reference cadence level, for example, the maximum allowable speed, rate, frequency, or period, an "above reference" signal **322** will be output. If the received body cadence information **310** is below the reference cadence level, for example, the maximum allowable speed, rate, frequency, or period, a "below" signal **324** will be output. On the other hand, if the received body cadence information **310** is equal to the reference cadence level, or within a reference range of preferred speed, rate, frequency, or period, an "equal" signal **326** will be output.

The controller **240** will depend on the outcome of the cadence comparison means decide on the mode of feedback signal to be delivered to a user.

In a preferred embodiment, the feedback signal is in the form of audio beats, for example, musical, drum, percussion, or vocal beats. Initially, audio beats of a pre-determined rhythm is stored or pre-stored in the data memory of the controller **240**. When the incoming body cadence information **310** has a cadence level above a prescribed reference level, for example, when the heart-rate, the running speed or the cycling cadence exceed a prescribed maximum, an "above" signal **322** will be sent to the controller **240**. In such a case, the controller will operate the audio processor **260** to reduce the beat rhythm of the feedback audio beats. By following the slower beat rhythm, a user will gradually reduce the heart-rate, speed or cadence to a desired or preferred level. If the incoming body cadence information **310** has a level which is above a prescribed reference level, for example, when the heart-rate, the running speed or the cycling cadence drops below a prescribed minimum, a "below" signal **324** will be sent to the controller **240**. In such a case, the controller will operate the audio processor 260 to increase the beat rhythm. By following the increased beat rhythm, a user will can gradually increase the heart-rate, speed or cadence to a desired or preferred level. The rhythm is increased or reduced gradually so that the transition from above the reference rate to the reference will be a smooth process.

On the other hand, if the incoming body cadence information is equal to or is within a prescribed range as set by the reference level, the controller will maintain the beat rhythm so that a user can maintain the appropriate cadence.

In addition or as an alternative, the controller can generate feedback output in different forms of physical stimulations. For example, by generating physical or mechanical beats or by generating small electrical pulses to stimulate a user. The physical stimulations can be standalone, intermittent or cyclical pulses. Where the physical stimulations are cyclical, the controller can cause the rhythm to vary in a similar manner as described above.

As a further alternative or a further addition, the feedback signal can be in the form of audible signals, such as vocal signals carrying numerical or other information. For example, the feedback signal can be a synthesised voice to inform the user of his/her instantaneous physical conditions or performance in numerical forms. For example, the information can be presented as, "speed-15 km per hour", "lap speed-26 seconds", "stroke frequency- 50 per minute", "hearrate- 126" etc. Alternatively, the audio signal can be for indicating preferred trends, for example, "faster", "slower", "maintain", "stop" or other appropriate information.

As a yet further alternative or a further addition, a background audio source can be integrally built into the cadence information processor of the controller **240** so that background audio signals, for example, music or songs, can be delivered to a user via a pair of ear phones. With such a background audio source, a user can be entertained with background music song while performing routine exercise. The performance feedback signal can be delivered by the controller upon interruption of the background audio broadcast or as by superimposition onto the background audio broadcast. A MP3 player or other digital audio players could be built in to provide background audio signals.

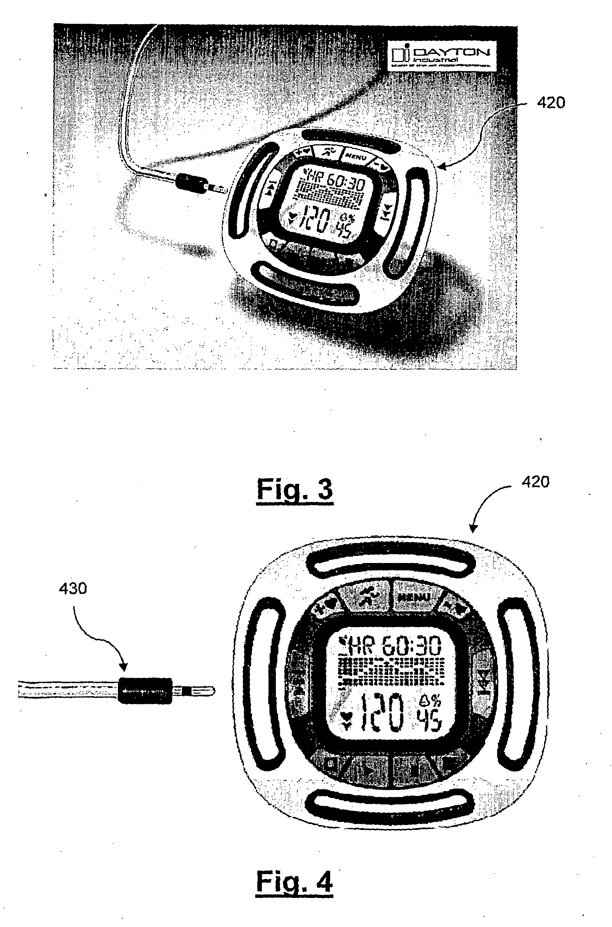

In addition to the physical feedback signals, a VDU is also provided on a preferred embodiment of this invention as shown in Figs. 3 and 4. In this preferred embodiment, relevant cadence information can be displayed on a display panel of the output device **420**. In addition, an earphone jack **430** is provided for output of the audio feedback signal. As an example, the heart-rate, percentage achievement of a user's heart-rate vis-à-vis the prescribed target heart rate as stored in the memory bank of the output device, are displayed on the VDU.

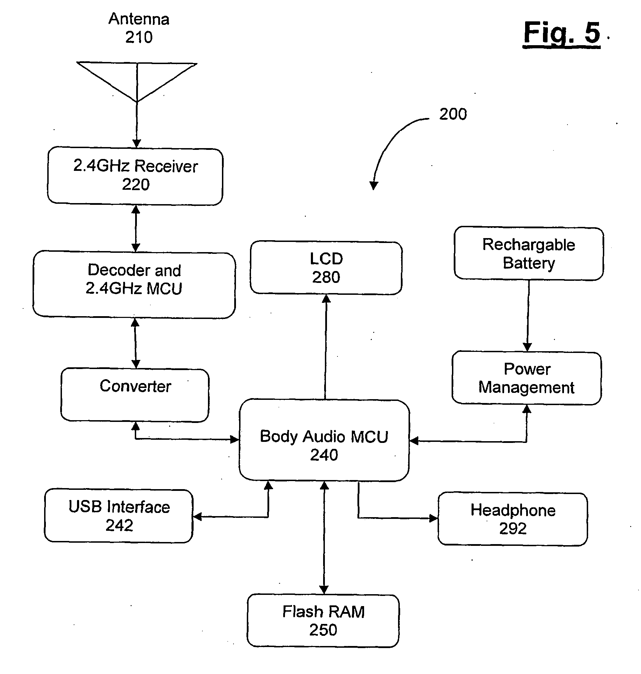

An exemplary circuit block diagram of a preferred embodiment of an output device is shown in Figs. 1 and 5. The output device comprises an antenna **210** which is connected to a wireless receiver **220** for operation at 2.4 GHz. The received wireless signal is forwarded to a demodulator and/or a decoder for conversion to baseband signals which can be readily processed by the controller **240**. The controller is further connected to a flash RAM **250**, a USB interface **242**, a VDU **280** and audio outputs **260**. The USB interface is for interfacing with an external USB device so that songs, music, beats or like can be downloaded and stored as an background audio source **260**. The flash RAM **250** provides storage means for storing the background audio source, as well as providing storage for other data, for example, user specific physical or physiological data or user preferred performance or reference levels. The VDU **280** is also provided so that a user can refer to the video display for an update on the instantaneous information, if the user so wishes. Power for operating the output device is obtained from a battery via a battery management unit.

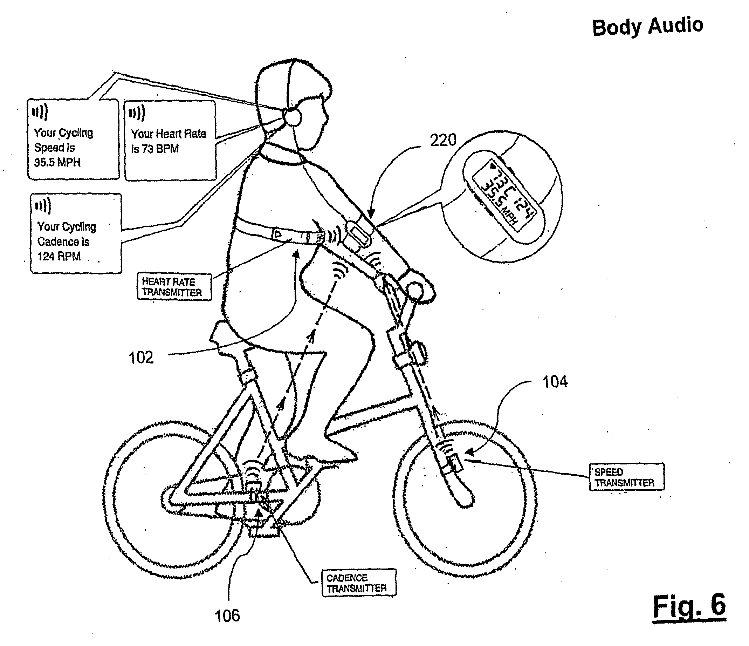

In Fig. 6, there is shown a first exemplary application of the monitoring apparatus of this invention. In this application, the monitoring apparatus is configured for monitoring the cycling cadence as well as the heart-rate of a user. In this application, two sensors are provided, namely, a heart-rate sensor **102** in the form of a chest strap and a cycling cadence transmitter **106** for monitoring the cycling cadence of a user. In addition, a speed transducer **104** is mounted onto the front wheel of the bicycle for transmitting speed information to the output device **220**. Thus, three sensors **102**, **104**, **106** are connected to the output device and the output device communicates with the sensors by, for example, time division multiplexed access (TDMA) protocols.

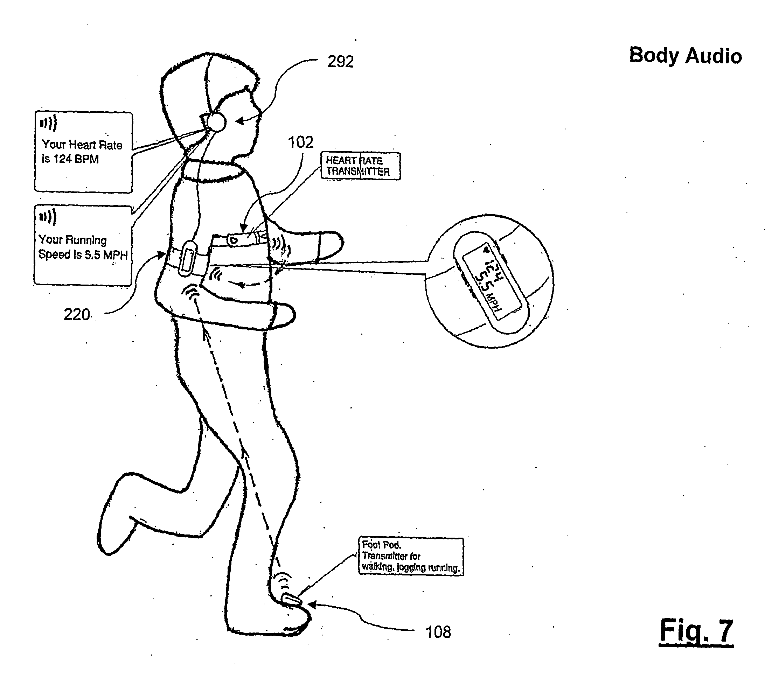

In Fig. 7, there is shown a second exemplary application of the monitoring apparatus of this invention. In this application, the cadence monitoring apparatus is configured for monitoring the running speed and heart-rate of a user. To cater for this application, a chest-strap type heart-rate monitoring sensor **102** and a foot-step sensor **108** are provided. Similar to the example of Fig. 6, the sensors communicate with the output device **220** by wireless communication links, for example, at 2.4 GHz. Upon receipt of the heart-rate and running speed body cadence information from the sensors, the output device will provide the user with audio feedback signals on the instantaneous performance, as shown in the dialogue boxes of Figs. 6 and 7.

In addition or as an alternative to the dialogue-type feedback, the controller may vary the rhythm of the background audio output by increasing the rhythm of the background music so as to urge a user to speed up, to reduce the rhythm of the background music or song so as to urge the user to slow down, or to maintain a constant musical beat to inform a user that the current cadence should be maintained.

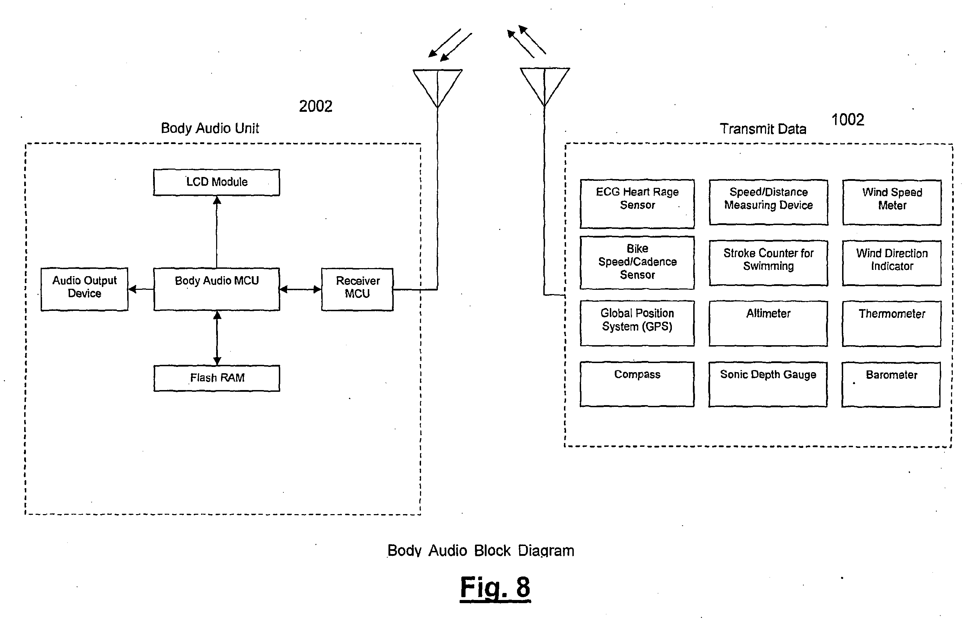

Fig. 8 depicts a further preferred embodiment of the present invention in which a plurality of sensors are schematically included. The sensor comprises an ECG heart-rate sensor, bicycle speed/cadence sensor, speed or distant measuring device, stroke counter for streaming etc. The sensors may be mounted separately on the body and connected via a wireless link to a wireless receiver in the output unit **2002** without loss of generality. In this preferred embodiment, the apparatus is configured as a sports performance monitoring apparatus comprising a plurality of exercise performance sensors in the sensing device **1002** and a signal processing unit. The exercise performance sensors may be one or more of the sensors as shown in the block diagram of Fig. 8. Specifically, the exercise performance sensors may be one or more of the following: ECG sensor, swimming sensor comprising arm-stroke counter and arm-span sensor, foot-stroke counter and foot-span sensor, cycling cadence sensor for monitoring exercise related signals.

In addition to sensors for monitoring performance signals, the exercise performance sensor may also include the following useful sensors such as compass, global positioning system (GPS) sensor, altimeter, sonic depth gauge, wind speed meter, wind direction indicator, thermometer, barometer or other useful sensors.

More particularly, the signal processing means is for processing exercise performance signals received from the exercise performance sensor to convert into useful information which can be processed by the controller. More specifically, the signal processing means comprises means for processing ECG pulses received from the sensor and transforms the received heart pulse signals into heart-rate information. Likewise, the signal processing means can transform foot-stroke frequency and foot-span information into running speed information, and/or can transform arm-stroke and arm-span information into lap counting or swimming speed. Similarly, the signal processing means can transform bicycle cadence information received from the cadence sensor into cycling speed and other relevant information. To mitigate shortcomings of conventional video display unit, the signal transformation unit further comprises means for converting the relevant information into audible signal for delivery to a user.

Thus, in this preferred embodiment, movement actuated performance signals such as heart pulses, foot-strokes, swimming strokes and/or cycling cadence are detected by the exercise performance sensors. Exercise performance signals thus generated are transmitted via wireless links to the signal processing means and are then forwarded to the user as feedback signals in the manner described above. Of course, wired connection may be made between the exercise performance sensor and the signal transformation unit without loss of generality.

While the present invention has been explained by reference to the examples or preferred embodiments described above, it will be appreciated that the embodiments are examples to assist understanding of the present invention and are not meant to be restrictive.

Furthermore, while the present invention has been explained by reference to a portable performance monitoring apparatus, it should be appreciated that the invention can apply, whether with or without modification, to other performance monitoring means without loss of generality.

## Claims

1. A monitoring apparatus for monitoring physical or physiological parameters and determining performance information of a person, said physical or physiological parameters including heart rate, pulse rate, swimming stroke, running tempo, cycling cadence, or the like, the monitoring apparatus comprising:-
a sensor for sensing physical or physiological parameters as performance information of a person,
signal processing means for converting performance information of a person into body cadence signals,
cadence comparison means for comparing at least one of said body cadence signals with at least one predetermined reference cadence level, and
control means for delivery of an output feedback signal to a user in response to said detected cadence signals,
wherein said output feedback signal is variable and dependent on the outcome of comparison between said detected cadence signals and said reference cadence level; **characterized in that** the output feedback signals comprise physical stimulations having a rhythm which is increased or decreased gradually to permit a user a smooth transition to reach said reference cadence level .

2. A monitoring apparatus according to Claim 1, further comprising a feedback signal source for generating said output feedback signal, wherein the contents and/or rhythm of said output feedback signal is dependent on the outcome of said cadence comparison means, and the physical stimulations comprise audio beats, mechanical beats or electrical pulses arranged to stimulate a user.

3. A monitoring apparatus according to Claims 1 or 2, wherein the physical stimulations are superimposed onto a background audio broadcast to a user, the background audio broadcast comprising music or songs.

4. A monitoring apparatus according to any preceding Claims,wherein the physical stimulations include vocal signals indicating preferred trends such as "faster", "slower", "maintain", "stop" or other appropriate information.

5. A monitoring apparatus according to any of the preceding Claims, wherein the rhythm of said audio output feedback signals is dependent on the outcome of comparison between said detected body cadence signals and said reference cadence level.

6. A monitoring apparatus according to any of the preceding Claims, wherein said output feedback signals further comprise audio information relating to instantaneous body cadence information of a person.

7. A monitoring apparatus according to any of the preceding Claims, wherein signals stored in said feedback signal source have a pre-determined rhythm, and wherein the rhythm of output feedback signals for delivery to a user being variable from said pre-determined rhythm such that the extent of variation of the rhythm of said feedback signals from said pre-determined rhythm is representative of the extent of departure of said body cadence signals from said preset or predetermined reference cadence level.

8. A monitoring apparatus according to any of the preceding Claims, wherein said cadence information comprises heart rate, foot-stroke frequency, hand- stroke frequency, lap time (swimming), running speed, and like body parametric performance information.

9. A monitoring apparatus according to any of the preceding Claims, wherein the instantaneous rhythm of said output feedback signals is decreased from said pre-determined rhythm to a lower rhythm when said detected cadence signals is above a threshold cadence level.

10. A monitoring apparatus according to any of the preceding Claims, wherein said feedback signal source comprise background audio output signals and cadence information audio output signals, said background audio output signals being delivered continuously to a user and said cadence information audio output signals being delivered to a user intermittently.

11. A monitoring apparatus according to Claim 10, wherein said cadence information audio output signals are delivered to a user at regular intervals, at pre-determined intervals and/or upon detection of a variation of the cadence information exceeding a pre-determined range.

12. A monitoring apparatus according to Claim 10, wherein said cadence information audio output signals are delivered when delivery of said background audio output is interrupted.

13. A monitoring apparatus according Claim 10, wherein delivery of background audio output signals and delivery of cadence information audio output signals share a common audio channel.

14. A monitoring apparatus according to Claims 10 to 13, wherein said background audio output comprises rhythmic audio signals including songs, music, temporal or rhythmic beats, or the like.

15. A monitoring apparatus according to Claim 14, wherein rhythm of said background audio output is varied according to the instantaneous body cadence information.

16. A monitoring apparatus according to any of the preceding Claims, wherein said feedback signals comprise audio delivery of body cadence information in numerical values.

17. A monitoring apparatus according to any of the preceding Claims, wherein said sensor is for sensing cadence parameters of a person which arise from physical exercise, and wherein said controller comprising processing means converts said cadence parameters into performance indicating parameters.

18. A monitoring apparatus according to Claim 17, wherein said performance indicating parameters includes heat rate, swimming speed, lap time, running speed, blood pressure, or like parameters.

19. A monitoring apparatus according to any of the preceding Claims, wherein said sensor is adapted for sensing at least one of the following parameters: stroke frequency (including foot-stroke frequency and arm-stroke frequency), stroke period (including foot-stroke period and arm-stroke period), pedalling frequency, pedalling speed, running speed.

20. A monitoring apparatus according to Claim 19, wherein the performance indicating parameters include running speed, swimming speed, cycling speed, expected time to complete a prescribed range or the like

21. A monitoring apparatus according to any of the preceding Claims, wherein the sensor is for sensing pedal frequency of a cyclist and the feedback signals comprises the numeric value at least one of the group including cycling cadence, cycling speed, remaining time-to-arrive or like.

22. A monitoring apparatus according to any of the preceding Claims, further comprising an ambient sensor for sensing at least one of the following ambient parameters: wind speed, wind direction, temperature, UV level, humidity; wherein said audio output signals further comprises at least one of the following numerical values- wind speed, wind direction, temperature, UV level, humidity

23. A monitoring apparatus according to any of the preceding Claims, wherein the sensor is for sensing step frequency of a runner and the audio output signals comprises at least one of the following numerical values- running cadence, running speed, remaining time-to-arrive or like information.

24. A monitoring apparatus according to any of the preceding Claims, further comprising storage means for storing at least one target cadence value or at least on target performance value, wherein said cadence comparison means comprises means for comparing detected cadence information against said target cadence value, and said controller generates an audio feedback to a user regarding the instantaneous performance of a user.

25. A monitoring apparatus according to Claim 24, wherein said audio feedback comprises musical beats indicative of the rhythm of exercise that a user is required to perform in order to achieve, reach or maintain said target cadence value or said target performance value.

26. A monitoring apparatus according to any of the preceding claims, wherein said feedback source comprises a background audio source for generating background audio signals, said background audio source comprising a radio receiver, a MP3 player, or other digital audio playback devices.

27. A monitoring apparatus according to any of the preceding claims, wherein said sensor is housed in a first housing and said audio source is housed in a second housing, said first and second housing being detached from each other, and said sensor and said control means being wirelessly linked.

28. A portable performance monitoring apparatus for monitoring performance conditions of a user during physical exercise comprising a monitoring apparatus according to any of the preceding Claims, wherein the sensor is adapted for detecting performance related cadence parameters of a person, and the output feedback signals are arranged to indicate a trend to stimulate a user to adjust his/her instantaneous physical activities in response to said output feedback signals to reach or maintain a performance target.

29. A performance monitoring apparatus according to Claim 28, wherein said physical feedback signal is delivered to a user by audio feedback.

30. A performance monitoring apparatus according to Claim 29, wherein said sensor is for sensing heart pulses and the audio feedback information comprises heart rate information of a user.

31. An apparatus according to any of the preceding Claims 28-30, further comprising wireless communication means for transmitting said detected performance related cadence information wireless.

## Patentansprüche

1. Überwachungsvorrichtung zum Überwachen physischer oder physiologischer Parameter und zum Bestimmen einer Leistungsinformation einer Person, wobei die physischen oder physiologischen Parameter eine Herzschlagrate, eine Pulsrate, einen Schwimmzug, ein Lauftempo, eine Radfahrkadenz oder Ähnliches umfassen, wobei die Überwachungsvorrichtung aufweist:
einen Sensor zum Erfassen physischer oder physiologischer Parameter als Leistungsinformation einer Person,
ein Signalverarbeitungsmittel zum Umwandeln von Leistungsinformation einer Person in Körperkadenzsignale,
ein Kadenzvergleichsmittel zum Vergleichen mindestens eines der Körperkadenzsignale mit mindestens einem vorbestimmten Kadenzreferenzpegel und
einem Steuermittel zum Bereitstellen eines Ausgangsrückkoppelsignals an einen Benutzer als Reaktion auf die erfassten Kadenzsignale,
wobei das Ausgangsrückkoppelsignal veränderbar ist und von dem Ergebnis des Vergleichs zwischen den erfassten Kadenzsignalen und dem Kadenzreferenzpegel abhängt,
dadurch gekenntzeichnet, dass die Ausgangsrückkoppelsignale physische Stimulationen umfassen mit einem Rhythmus, der stufenweise erhöht oder verringert wird, sodass einem Benutzer ein glatter Übergang ermöglicht wird, um den Kadenzreferenzpegel zu erreichen.

2. Überwachungsvorrichtung nach Anspruch 1, darüber hinaus mit einer Rückkoppelsignalquelle zum Erzeugen des Ausgangsrückkoppelsignals, wobei der Inhalt und/oder der Rhythmus des Ausgangsrückkoppelsignals von dem Ergebnis des Kadenzvergleichsmittels abhängt und die physischen Stimulationen Audiorhythmen, mechanische Rhythmen oder elektrische Impulse, die so eingerichtet sind, dass sie einen Benutzer stimulieren, umfassen.

3. Überwachungsvorrichtung nach Anspruch 1 oder 2, wobei die physischen Stimulationen einer Hintergrundaudioausstrahlung an einen Benutzer überlagert sind, wobei die Hintergrundaudioausstrahlung Musik oder Lieder umfasst.

4. Überwachungsvorrichtung nach einem der vorhergehenden Ansprüche, wobei die physischen Stimulationen Stimmensignale umfassen, die bevorzugte Tendenzen, wie zum Beispiel "schneller", "langsamer", "beibhalten", "anhalten" oder andere passende Informationen umfassen.

5. Überwachungsvorrichtung nach einem der vorhergehenden Ansprüche, wobei der Rhythmus der Audioausgangsrückkoppelsignale von dem Ergebnis des Vergleichs zwischen den erfassten Körperkadenzsignalen und dem Kadenzreferenzpegel abhängt.

6. Überwachungsvorrichtung nach einem der vorhergehenden Ansprüche, wobei die Ausgangsrückkoppelsignale darüber hinaus Audioinformationen umfassen, die die momentane Körperkadenzinformation einer Person betreffen.

7. Überwachungsvorrichtung nach einem der vorhergehenden Ansprüche, wobei in der Rückkoppelsignalquelle gespeicherte Signale einen vorbestimmten Rhythmus aufweisen und wobei der Rhythmus der Ausgangsrückkoppelsignale zur Bereitstellung an einen Benutzer gegenüber dem vorbestimmten Rhythmus veränderbar sind, sodass der Grad an Veränderung des Rhythmus des Rückkoppelsignals gegenüber dem vorbestimmten Rhythmus den Grad an Abweichung der Körperkadenzsignale von dem voreingestellten oder vorbestimmten Kadenzreferenzpegel widerspiegelt.

8. Überwachungsvorrichtung nach einem der vorhergehenden Ansprüche, wobei die Kadenzinformation eine Herzschlagrate, eine Fußschlagfrequenz, eine Handschlagfrequenz, eine Rundenzeit (Schwimmen), eine Laufgeschwindigkeit und ähnliche parametrische Körperleistungsinformation umfasst.

9. Überwachungsvorrichtung nach einem der vorhergehenden Ansprüche, wobei der momentane Rhythmus der Ausgangsrückkoppelsignale gegenüber dem vorbestimmten Rhythmus zu einem geringeren Rhythmus verringert wird, wenn die erfassten Kadenzsignale über einem Kadenzschwellenpegel liegen.

10. Überwachungsvorrichtung nach einem der vorhergehenden Ansprüche, wobei die Rückkoppelsignalquelle Hintergrundaudioausgangssignale und Kadenzinformationsaudioausgangssignale umfasst, wobei die Hintergrundaudioausgangssignale kontinuierlich an einen Benutzer bereitgestellt werden und die Kadenzinformationsaudioausgangssignale intermittierend an einen Benutzer bereitgesiellt werden.

11. Überwachungsvorrichtung nach Anspruch 10, wobei die Kadenzinformaiionsaudioausgangssignale an einen Benutzer in gleichmäßigen Intervallen, in vorbestimmten Intervallen und/oder bei Erfassung einer Änderung der Kadenzinformation, die einen vorbestimmten Bereich übersteigt, bereitgestellt werden.

12. Überwachungsvorrichtung nach Anspruch 10, wobei die Kadenzinformationsaudioausgangssignale bereitgestellt werden, wenn eine Bereitstellung des Hintergrundaudioausgangs unterbrochen ist.

13. Überwachungsvorrichtung nach Anspruch 10, wobei eine Bereitstellung der Hintergrundaudioausgangssignale und eine Bereitstellung von Kadenzinformationsaudioausgangssignalen einen gemeinsamen Audiokanal teilen.

14. Überwachungsvorrichtung nach einem der Ansprüche 10 bis 13, wobei der Hintergrundaudioausgang rhythmische Audiosignale einschließlich Liedern, Musik, zeitlichen oder rhythmischen Schlägen oder Ähnliches umfasst.

15. Überwachungsvorrichtung nach Anspruch 14, wobei der Rhythmus des Hintergrundaudioausgangs gemäß der momentanen Körperkadenzinformation verändert wird.

16. Überwachungsvorrichtung nach einem der vorhergehenden Ansprüche, wobei die Rückkoppelsignale eine Audiobereitstellung von Körperkadenzinformation in numerischen Werten umfasst.

17. Überwachungsvorrichtung nach einem der vorhergehenden Ansprüche, wobei der Sensor zum Erfassen von Kadenzparametern einer Person geeignet ist, die aus körperlicher Bewegung resultieren und wobei die Steuerung mit einem Verarbeitungsmittel die Kadenzparameter in Leistungsanzeigeparameter umwandelt.

18. Überwachungsvorrichtung nach Anspruch 17, wobei die Leistungsanzeigeparameter eine Herzschlagrate, eine Schwimmgeschwindigkeit, eine Rundenzeit, eine Laufzeit, Blutdruck oder ähnliche Parameter umfassen.

19. Überwachungsvorrichtung nach einem der vorhergehenden Ansprüche, wobei der Sensor zum Erfassen mindestens einer der folgenden Parameter eingerichtet ist: Schlagzahl (einschließlich Fußschlagzahl und Armschlagzahl), Schlagperiode (einschließlich Fußschlagperiode und Armschtagperiode), Trittfrequenz, Trittgeschwindigkeit, Laufgeschwindigkeit.

20. Überwachungsvorrichtung nach Anspruch 19, wobei die Leistungsanzeigeparameter eine Laufgeschwindigkeit, eine Schwimmgeschwindigkeit, eine Radfahrgeschwindigkeit, eine erwartete Zeit, um einen vorbestimmten Bereich zu vollenden oder Ähnliches umfassen.

21. Überwachungsvorrichtung nach einem der vorhergehenden Ansprüche, wobei der Sensor zum Erfassen einer Pedalfrequenz eines Radfahrers dient und die Rückkoppelsignale den numerischen Wert mindestens einer aus der Gruppe aufweist, die eine Radfahrkadenz, eine Radfahrgeschwindigkeit, die verbleibende Zeit bis zur Ankunft oder Ähnliches, umfasst.

22. Überwachungsvorrichtung nach einem der vorhergehenden Ansprüche, darüber hinaus mit einem Umgebungssensor zum Erfassen mindestens eines der folgenden Parameter: Windgeschwindigkeit, Windrichtung, Temperatur, UV-Pegel, Luftfeuchtigkeit, wobei die Audioausgangssignale darüber hinaus mindestens einen der folgenden numerischen Werte umfassen: Windgeschwindigkeit, Windrichtung, Temperatur, UV-Pegel, Luftfeuchtigkeit.

23. Überwachungsvorrichtung nach einem der vorhergehenden Ansprüche, wobei der Sensor zum Erfassen einer Schrittfrequenz eines Läufers dient und die Audioausgangssignale mindestens einen der folgenden numerischen Werte umfasst: Laufkadenz, Laufgeschwindigkeit, verbleibende Zeit bis zur Ankunft oder ähnliche Information.

24. Überwachungsvorrichtung nach einem der vorhergehenden Ansprüche, darüber hinaus mit einem Speichermittel zum Speichern mindestens eines Zielkadenzwerts oder mindestens eines Zielleistungswerts, wobei das Kadenzvergleichsmittel ein Mittel zum Vergleichen erfasster Kadenzinformation mit dem Zielkadenzwert umfasst und die Steuerung eine Audiorückkopplung an einen Benutzer erzeugt, die die momentane Leistung eines Benutzers betrifft.

25. Überwachungsvorrichtung nach Anspruch 24, wobei die Audiorückkopplung musikalischen Takt, der den Takt der Übung umfasst, die ein Benutzer ausführen muss, um den Zielkadenzwert oder den Zielleistungswert zu erreichen oder zu erhalten.

26. Überwachungsvorrichtung nach einem der vorhergehenden Ansprüche, wobei die Rückkoppelquelle eine Hintergrundaudioquelle zum Erzeugen von Hintergrundaudiosignalen aufweist, wobei die Hintergrundaudioquelle einen Radioempfänger, einen MP3-Spieler oder andere digitale Audioabspieieinrichtungen aufweist.

27. Überwachungsvorrichtung nach einem der vorhergehenden Ansprüche, wobei der Sensor in einem ersten Gehäuse aufgenommen ist und die Audioquelle in einem zweiten Gehäuse aufgenommen ist, wobei die ersten und zweiten Gehäuse voneinander abgenommen sind und der Sensor und das Steuermittel drahtlos verbunden sind.

28. Tragbare Leistungsüberwachungsvorrichtung zum Überwachen von Leistungszuständen eines Benutzers während körperlicher Übung mit einer Überwachungsvorrichtung nach einem der vorhergehenden Ansprüche, wobei der Sensor zum Erfassen von leistungsbezogenen Kadenzparametern einer Person eingerichtet ist und die Ausgangsrückkoppelsignale so eingerichtet sind, dass sie eine Tendenz anzeigen, um einen Benutzer zu stimulieren, seine momentanen körperliche Aktivitäten als Reaktion auf die Ausgangsrückkoppelsignale anzupassen, sodass er eine Zielleistung erreicht oder beibehält.

29. Leistungsüberwachungsvorrichtung nach Anspruch 28, wobei das physische Rückkoppelsignal durch Audiorückkopplung an einen Benutzer bereitgestellt wird.

30. Leistungsüberwachungsvorrichtung nach Anspruch 29, wobei der Sensor zum Erfassen von Herzschlagimpulsen dient und die Audiorückkoppelinformation eine Herzschlagrateinformation eines Benutzers aufweist.

31. Vorrichtung nach einem der Ansprüche 28 bis 30, darüber hinaus mit einem drahtlosen Kommunikationsmittel zum drahtlosen Übertragen der die erfasste Leistung betreffenden Kadenzinformation.

## Revendications

1. Appareil de surveillance destiné à surveiller des paramètres physiques ou physiologiques et à déterminer les informations de performance d'une personne, lesdits paramètres physiques ou physiologiques comprenant le rythme cardiaque, la fréquence du pouls, le mouvement de nage, le rythme de course, la cadence de pédalage ou similaire, l'appareil de surveillance comprenant:
- un capteur destiné à détecter les paramètres physiques ou physiologiques en tant qu'informations de performance d'une personne,
- des moyens de traitement de signaux destinés à convertir les informations de performance d'une personne en signaux de cadence corporelle,
- des moyens de comparaison de cadence destinés à comparer au moins un desdits signaux de cadence corporelle avec au moins un niveau de cadence de référence prédéterminé, et
- des moyens de contrôle destinés à délivrer un signal de retour de sortie à un utilisateur en réponse auxdits signaux de cadence détectés,
- dans lequel ledit signal de retour de sortie est variable et dépendant du résultat de comparaison entre lesdits signaux de cadence détectés et ledit niveau de cadence de référence ; **caractérisé en ce que** les signaux de retour de sortie comprennent des stimulations physiques ayant un rythme qui est augmenté ou diminué progressivement pour permettre à un utilisateur une transition en douceur pour atteindre ledit niveau de cadence de référence.

2. Appareil de surveillance selon la revendication 1, comprenant en outre une source de signal de retour destinée à générer ledit signal de retour de sortie, dans lequel le contenu et/ou le rythme dudit signal de retour de sortie dépend du résultat desdits moyens de comparaison de cadence, et les stimulations physiques comprennent des battements audio, des battements mécaniques ou des impulsions électriques destinées à stimuler un utilisateur.

3. Appareil de surveillance selon la revendication 1 ou 2, dans lequel les stimulations physiques sont superposées à une diffusion audio de fond destinée à un utilisateur, la diffusion audio de fond comprenant de la musique ou des chansons.

4. Appareil de surveillance selon l'une quelconque des revendications précédentes, dans lequel les stimulations physiques comprennent des signaux vocaux indiquant des tendances préférées telles que « plus vite », « plus lestement », « maintenir », « arrêter » ou d'autres informations appropriées.

5. Appareil de surveillance selon l'une quelconque des revendications précédentes, dans lequel le rythme desdits signaux de retour de sortie audio dépend du résultat de la comparaison entre lesdits signaux de cadence corporelle détectés et ledit niveau de cadence de référence.

6. Appareil de surveillance selon l'une quelconque des revendications précédentes, dans lequel lesdits signaux de retour de sortie comprennent en outre des informations audio concernant les informations de cadence corporelle instantanées d'une personne.

7. Appareil de surveillance selon l'une quelconque des revendications précédentes, dans lequel les signaux stockés dans ladite source de signaux de retour ont un rythme prédéterminé, et le rythme des signaux de retour de sortie destinés à être délivrés à un utilisateur est variable par rapport audit rythme prédéterminé de telle sorte que l'importance de la variation du rythme desdits signaux de retour par rapport audit rythme prédéterminé est représentative de l'importance de la déviation desdits signaux de cadence corporelle par rapport audit niveau de cadence de référence prédéfini ou prédéterminé.

8. Appareil de surveillance selon l'une quelconque des revendications précédentes, dans lequel lesdites informations de cadence comprennent le rythme cardiaque, la fréquence de mouvement des pieds, la fréquence de mouvement des mains, le temps intermédiaire (natation), la vitesse de course, et des informations de performance paramétriques corporelles similaires.

9. Appareil de surveillance selon l'une quelconque des revendications précédentes, dans lequel le rythme instantané desdits signaux de retour de sortie est réduit dudit rythme prédéterminé à un rythme inférieur lorsque lesdits signaux de cadence détectés sont au-dessus d'un niveau de cadence de seuil.

10. Appareil de surveillance selon l'une quelconque des revendications précédentes, dans lequel ladite source de signaux de retour comprend des signaux de sortie audio de fond et des signaux de sortie audio d'informations de cadence, lesdits signaux de sortie audio de fond étant délivrés en continu à un utilisateur et lesdits signaux de sortie audio d'informations de cadence étant délivrés à un utilisateur de façon intermittente.

11. Appareil de surveillance selon la revendication 10, dans lequel lesdits signaux de sortie audio d'informations de cadence sont transmis à un utilisateur à intervalles réguliers, à intervalles prédéterminées et/ou lors de la détection d'une variation des informations de cadence dépassant une plage prédéterminée.

12. Appareil de surveillance selon la revendication 10, dans lequel lesdits signaux de sortie audio d'informations de cadence sont délivrés lorsque la délivrance de ladite sortie audio de fond est interrompue.

13. Appareil de surveillance selon la revendication 10, dans lequel la délivrance des signaux de sortie audio de fond et la délivrance des signaux de sortie audio d'informations de cadence partageant un canal audio commun.

14. Appareil de surveillance selon les revendications 10 à 13, dans lequel ladite sortie audio de fond comprend des signaux audio rythmiques comprenant des chansons, de la musique, des battements temporels ou rythmiques, ou similaires.

15. Appareil de surveillance selon la revendication 14, dans lequel le rythme de ladite sortie audio de fond varie en fonction des informations de cadence corporelle instantanées.

16. Appareil de surveillance selon l'une quelconque des revendications précédentes, dans lequel lesdits signaux de retour comprennent une délivrance audio des informations de cadence corporelle sous forme de valeurs numériques.

17. Appareil de surveillance selon l'une quelconque des revendications précédentes, dans lequel ledit capteur est destiné à détecter les paramètres de cadence d'une personne qui découlent d'un exercice physique, et dans lequel ledit contrôleur comprenant des moyens de traitement convertit lesdits paramètres de cadence en paramètres d'indication de performance.

18. Appareil de surveillance selon la revendication 17, dans lequel lesdits paramètres d'indication de performance comprennent la fréquence cardiaque, la vitesse de nage, le temps intermédiaire, la vitesse de course, la tension artérielle, ou des paramètres similaires.

19. Appareil de surveillance selon l'une quelconque des revendications précédentes, dans lequel ledit capteur est destiné à détecter au moins l'un des paramètres suivants : fréquence de mouvement (comprenant fréquence de mouvement des pieds et fréquence de mouvement des bras), période de mouvement (comprenant période de mouvement des pieds et période de mouvement des bras), fréquence de pédalage, vitesse de pédalage, vitesse de course.

20. Appareil de surveillance selon la revendication 19, dans lequel les paramètres d'indication de performance comprennent la vitesse de course, la vitesse de nage, la vitesse de pédalage, le temps prévu pour parcourir une distance prévue ou similaire.

21. Appareil de surveillance selon l'une quelconque des revendications précédentes, dans lequel le capteur est conçu pour détecter une fréquence de pédalage d'un cycliste et les signaux de retour comprennent la valeur numérique d'au moins un élément du groupe comprenant la cadence de pédalage, la vitesse de pédalage, le temps restant jusqu'à l'arrivée ou similaire.

22. Appareil de surveillance selon l'une quelconque des revendications précédentes, comprenant en outre un capteur ambiant destinée à détecter au moins un des paramètres ambiants suivants : vitesse du vent, direction du vent, température, niveau d'UV, humidité ; dans lequel lesdits signaux de sortie audio comprennent en outre au moins une des valeurs numériques suivantes - vitesse du vent, direction du vent, température, niveau d'UV, humidité.

23. Appareil de surveillance selon l'une quelconque des revendications précédentes, dans lequel le capteur est conçu pour détecter la fréquence de pas d'un coureur et les signaux de sortie audio comprennent au moins l'une des valeurs numériques suivantes - cadence de course, vitesse de course, temps restant jusqu'à l'arrivée ou informations similaires.

24. Appareil de surveillance selon l'une quelconque des revendications précédentes, comprenant en outre des moyens de stockage pour stocker au moins une valeur de cadence cible ou au moins une valeur de performance cible, dans lequel lesdits moyens de comparaison de cadence comprennent des moyens pour comparer des informations de cadence détectées à ladite valeur de cadence cible, et ledit contrôleur génère un retour audio destiné à un utilisateur concernant la performance instantanée d'un utilisateur.

25. Appareil de surveillance selon la revendication 24, dans lequel ledit retour audio comprend des battements musicaux indicatifs du rythme d'exercice qu'un utilisateur doit effectuer afin de réaliser d'atteindre ou de maintenir ladite valeur de cadence cible ou ladite valeur de performance cible.

26. Appareil de surveillance selon l'une quelconque des revendications précédentes, dans lequel ladite source de retour comprend une source audio de fond destinée à générer des signaux audio de fond, ladite source audio de fond comprenant un récepteur radio, un lecteur MP3, ou autres dispositifs de lecture audio numériques.

27. Appareil de surveillance selon l'une quelconque des revendications précédentes, dans lequel ledit capteur est logé dans un premier logement et ladite source audio est logée dans un second logement, lesdits premier et second logements étant détachés l'un de l'autre, et ledit capteur et lesdits moyens de contrôle étant reliés sans fil.

28. Appareil de surveillance de performance portable destiné à surveiller les conditions de performance d'un utilisateur au cours d'un exercice physique comprenant un appareil de surveillance selon l'une quelconque des revendications précédentes, dans lequel le capteur est conçu pour détecter des paramètres de cadence relatifs à la performance d'une personne, et les signaux de retour de sortie sont destinés à indiquer une tendance pour stimuler un utilisateur afin qu'il ajuste ses activités physiques instantanées en réponse auxdits signaux de retour de sortie pour atteindre ou maintenir une cible de performance.

29. Appareil de surveillance de performance selon la revendication 28, dans lequel ledit signal de retour physiques est délivré à un utilisateur par retour audio.

30. Appareil de surveillance de performance selon la revendication 29, dans lequel ledit capteur est conçu pour détecter les pulsations cardiaques et les informations de retour audio comprennent les informations de rythme cardiaque d'un utilisateur.

31. Appareil selon l'une quelconque des revendications précédentes 28 à 30, comprenant en outre des moyens de communication sans fil pour transmettre lesdites informations de cadence relatives à la performance détectées sans fil.
